(19) Europäisches Patentamt
European Patent Office
Office européen des brevets

(11) Veröffentlichungsnummer: 0 417 044 A2

# (12) EUROPÄISCHE PATENTANMELDUNG

(21) Anmeldenummer: 90810654.5

(22) Anmeldetag: 29.08.90

(51) Int. Cl.5: C07D 263/20, A01N 43/76, C07D 263/16

(30) Priorität: 07.09.89 CH 3248/89
07.06.90 CH 1905/90

(43) Veröffentlichungstag der Anmeldung: 13.03.91 Patentblatt 91/11

(84) Benannte Vertragsstaaten: AT BE CH DE DK ES FR GB GR IT LI LU NL SE

(71) Anmelder: CIBA-GEIGY AG
Klybeckstrasse 141
CH-4002 Basel(CH)

(72) Erfinder: Lang, Robert Werner, Dr.
Hagenbachweg 10
CH-4133 Pratteln(CH)
Erfinder: Ramos, Gerardo, Dr.
General Guisan-Strasse 39
CH-4144 Arlesheim(CH)

## (54) Neue Oxazoldinone und -thione als Pflanzenregulatoren.

(57) 3-Phenyl-oxazolidin-2-on- und -2-thion-5-carbonsäurederivate der untenstehenden Formel I in allen stereoisomeren Formen sind zur Regulierung des Pflanzenwachstums geeignet.

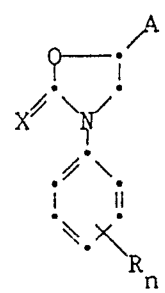

(I)

In dieser Formel stehen X für Sauerstoff oder Schwefel;
A für $-COOR_1$, $-COSR_1$, $-COO^{\ominus}M^{\oplus}$, $-CONR_2R_3$ oder -COCl;
R für Wasserstoff, Halogen oder Mono-, Di- oder Trihalomethyl;
n für 1, 2 oder 3;
$R_1$ für Wasserstoff, $C_1$-$C_4$-Alkyl, $C_2$-$C_6$-Alkenyl oder $C_2$-$C_6$-Alkinyl;
$R_2$ und $R_3$ unabhängig voneinander für Wasserstoff, $C_1$-$C_4$-Alkyl, $C_2$-$C_6$-Alkenyl, $C_2$-$C_6$-Alkinyl, $C_3$-$C_7$-Cycloalkyl, Aryl oder Heterocyclyl; oder $R_2$ und $R_3$ zusammen mit dem Stickstoffatom, an welches sie gebunden sind, für einen gegebenenfalls bis zu dreifach durch $C_1$-$C_4$-Alkyl oder $C_1$-$C_4$-Alkoxy substituierten gesättigten $C_3$-$C_7$-Heterocyclus stehen; und $M^{\oplus}$ das Aequivalent eines Alkali- oder Erdalkalimetallkations oder $HN^{\oplus}(R_2)_3$.
Die Erfindung umfasst die Herstellung und Anwendung dieser Verbindungen.

EP 0 417 044 A2

## NEUE OXAZOLIDINONE UND -THIONE ALS PFLANZENREGULATOREN

Die vorliegende Erfindung betrifft neue 3-Phenyl-oxazolidin-2-on- und -2-thion-5-carbonsäurederivate in allen stereoisomeren Formen, Verfahren zu deren Herstellung, diese Derivate als Wirkstoffe enthaltende Mittel als Pflanzenwuchsregulatoren sowie die Verwendung dieser Derivate oder der sie enthaltenden Mittel zur Regulierung des Pflanzenwachstums.

Die US-Patentschrift Nr. 4,013,445 offenbart 1-(Bis-trifluormethylphenyl)-2-oxo-pyrrolidin-4-carbonsäurederivate als Herbizide und zur Regulierung des Pflanzenwachstums.

Aus der französischen Patentschrift 1.363.615 und der US Patentschrift 3.136.620 ist die Verwendung von 1-Phenyl-2-oxo-pyrrolidin-4-carbonsäuren sowie deren Derivate, die am Phenylkern unsubstituiert oder durch Halogenatome und/oder eine Trifluormethylgruppe substituiert sind, als Aktivsubstanzen zur Beeinflussung des Pflanzenwachstums bekannt.

Es wurde nun gefunden, dass die erfindungsgemässen 3-Phenyl-oxazolidin-2-on- und -2-thion-5-carbonsäurederivate in allen stereoisomeren Formen sehr gute pflanzenwuchsregulierende Eigenschaften aufweisen.

Die erfindungsgemäss vorgeschlagenen 3-Phenyl-oxazolidin-2-on- und -2-thion-5-carbonsäurederivate entsprechen der Formel I

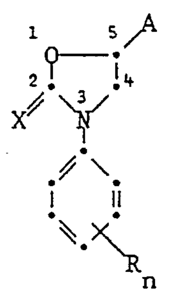

(I)

worin X für Sauerstoff oder Schwefel;
A für $-COOR_1$, $-COSR_1$, $-COO^{\ominus}M^{\oplus}$, $-CONR_2R_3$ oder $-COCl$;
R für Wasserstoff, Halogen, Mono-, Di- oder Trihalomethyl;
n für 1, 2 oder 3;
$R_1$ für Wasserstoff, $C_1$-$C_4$-Alkyl, $C_2$-$C_6$-Alkenyl oder $C_2$-$C_6$-Alkinyl;
$R_2$ und $R_3$ unabhängig voneinander für Wasserstoff, $C_1$-$C_4$-Alkyl, $C_2$-$C_6$-Alkenyl, $C_2$-$C_6$-Alkinyl, $C_3$-$C_7$-Cycloalkyl, Aryl oder Heterocyclyl; oder $R_2$ und $R_3$ zusammen mit dem Stickstoffatom, an welches sie gebunden sind, für einen gegebenenfalls bis zu dreifach durch $C_1$-$C_4$-Alkyl oder $C_1$-$C_4$-Alkoxy substituierten gesättigten 3- bis 7-gliedrigen Heterocyclus, der noch ein weiteres Heteroatom ausgewählt aus der Gruppe O, N und S enthalten kann, stehen; und $M^{\oplus}$ das Aequivalent eines Alkali- oder Erdalkalimetallkations oder $HN^{\oplus}(R_2)_3$ bedeutet sowie deren optisch aktiven Isomeren.

In diesen Definitionen steht Halogen für Fluor, Chlor, Brom oder Jod.

Unter Alkyl ist geradkettiges oder verzweigtes Alkyl zu verstehen, z.B. Methyl, Ethyl, n-Propyl, i-Propyl, n-Butyl, i-Butyl, sec.-Butyl oder tert. Butyl.

Unter Alkenyl ist geradkettiges oder verzweigtes Alkenyl zu verstehen; wie zum Beispiel: Vinyl, Allyl, 2-Propenyl, Methallyl, 3-Butenyl, 2-Butenyl, 3-Pentenyl, 2-Methyl-4-pentenyl, 3-Hexenyl.

In den Definitionen ist unter Alkinyl geradkettiges oder verzweigtes Alkinyl zu verstehen; z.B.: Propargyl, Aethinyl, 2-Propinyl, 3-Butinyl, 2-Methyl-3-pentinyl, 1,2-Dimethyl-3-butinyl.

Cycloalkyl steht beispielsweise für Cyclopropyl, Dimethylcyclopropyl, Cyclobutyl, Cyclopentyl, Methylcyclopentyl, Cyclohexyl oder Cycloheptyl, vorzugsweise aber für Cyclopropyl, Cyclopentyl oder Cyclohexyl.

Als Kationen $M^{\oplus}$ kommen insbesondere die von Alkalimetallen wie beispielsweise Lithium, Natrium oder Kalium und die von Erdalkalimetallen wie beispielsweise Magnesium oder Calcium in Betracht. Insbesondere steht $M^{\oplus}$ für $HN^{\oplus}(R_2)_3$, wobei die Substituenten $R_2$ gleich oder verschieden sein können, oder für die Kationen von Natrium oder Kalium.

Wenn A den Amidrest $-CONR_2R_3$ bedeutet, sind insbesondere die nachstehend genannten Reste bevorzugt: $-CONH_2$, $-CON(CH_3)_2$, $CON(C_2H_5)_2$,

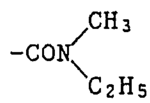 , 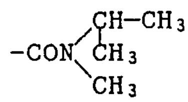 , 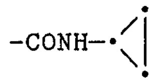 , 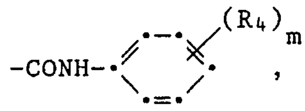 ,

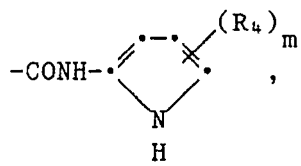 , 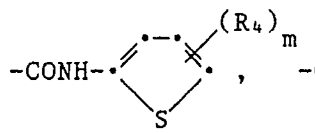 , 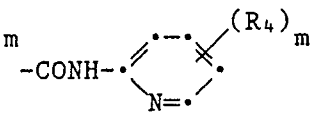 ,

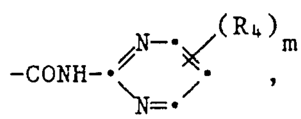 , 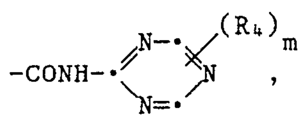 , 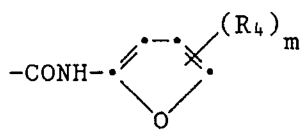

wobei für $R_4$ Halogen, $C_1$-$C_3$-Alkyl, $C_1$-$C_3$-Alkoxy; $C_1$-$C_3$-Halogenalkoxy und für m 0, 1, 2 oder 3 steht.

Beispiele für durch die Substituenten $R_2$ und $R_3$ allein oder gemeinsam mit dem sie tragenden Stickstoffatom gebildeten Heterocyclen sind Pyrrolidin, Pyrrol, Imidazol, Pyrazol, Piperazin, Piperidin, Morpholin, Pyridin, Pyrimidin, Thiazol, Thiomorpholin, Triazine und Triazole.

Beispiele für Aryl sind Phenyl und Naphthyl, welche Reste unsubstituiert oder bis zu viermal durch Halogen, Alkyl, Alkoxy, Alkylthio, Haloalkyl, Haloalkoxy, Nitro, Cyan, Carboxyl, Carbamoyl, Alkoxycarbonyl, Alkylcarbonyl, Alkylsulfonyl, Sulfamoyl und Alkylsulfamoyl substitutiert sind.

Bevorzugte Verbindungen der Formel I sind jene, in denen A für $-COOR_1$ oder $-COO^{\ominus}M^{\oplus}$ steht. Aus dieser Gruppe sind als Einzelverbindungen zu nennen: 3-(3,5-Bis-trifluormethylphenyl)-oxazolidin-2-on-5-carbonsäure,
3-(3,5-Bis-trifluormethylphenyl)-oxazolidin-2-on-5-carbonsäuremethylester und
3-(3,5-Bis-trifluormethylphenyl)-oxazolidin-2-on-5-carbonsäuretributylammoniumsalz.

Besonders gut wirkende Gruppen von Verbindungen der Formel I bilden die optisch aktiven Verbindungen der Formeln (R)-I und (S)-I

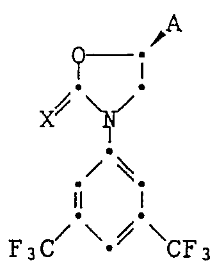 (R)-(I) 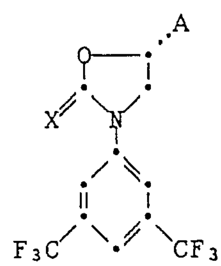 (S)-(I)

worin A und X die unter Formel I angegebene Bedeutung haben. Aus dieser Gruppe fallen durch ihre gute biologische Wirkung jene Verbindungen ins Auge, bei denen A für $-COOR_1$ oder $-COO^{\ominus}M^{\oplus}$ steht. Als bevorzugte Einzelverbindungen der Formeln (R)-I und (S)-I sind zu nennen:
5-(R)-3-(3,5-Bis-trifluormethylphenyl)-oxazolidin-2-on-5-carbonsäure
5-(R)-3-(3,5-Bis-trifluormethylphenyl)-oxazolidin-2-thion-5-carbonsäuremethylester,
5-(S)-3-(3,5-Bis-trifluormethylphenyl)-oxazolidin-2-on-5-carbonsäure und
5-(S)-3-(3,5-Bis-trifluormethylphenyl)-oxazolidin-2-thion-5-carbonsäuremethylester.

Die erfindungsgemässen Verbindungen der Formel I können auf an sich bekannte Weise hergestellt werden, indem man, im Falle der racemischen Verbindungen der Formel I, ein Anilin der Formel II

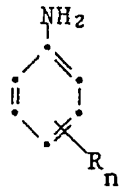

(II)

worin n und R die unter der Formel I gegebene Bedeutung haben, mit racemischem Glycidol ((±)-2,3-Epoxy-1-propanol) der Formel III

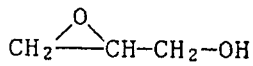

(III)

zum 1,2-Propandiolanilid der Formel IV umsetzt,

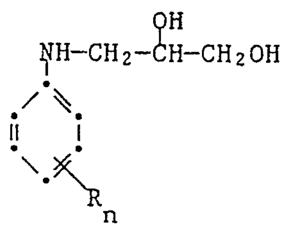

(IV)

worin n und R die unter der Formel I gegebene Bedeutung haben, dieses Diol dann in einem geeigneten Lösungsmittel, in Gegenwart einer Base mit Diethylcarbonat zum 3-Phenyl-5-hydroxymethyloxazolidin-2-on der Formel V cyclisiert,

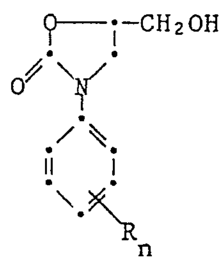

(V)

worin n und R die unter der Formel I gegebene Bedeutung haben, diese Verbindung dann mit einem oxidierenden Mittel zur 3-Phenyl-oxazolidin-2-on-5-carbonsäure der Formel Ia oxidiert,

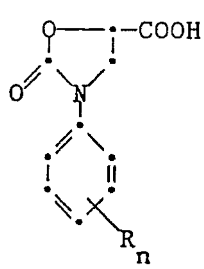

(Ia)

worin n und R die unter der Formel I gegebene Bedeutung haben, und diese Säure gewünschtenfalls in an sich bekannter Weise zum Salz oder durch Umsetzen mit einer Verbindung der Formel VI

H-B (VI)

worin B das Radikal eines Alkohols, Mercaptans oder Amins bedeutet, zum Ester, Thioester oder Amid der Formel Ib umwandelt,

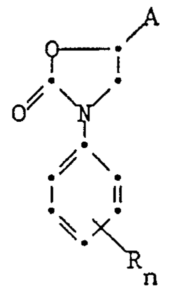

(Ib)

worin A, n und R die unter der Formel I gegebene Bedeutung haben.

Ein Ester der Formel Ic

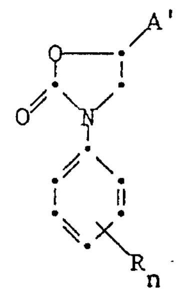

(Ic)

worin A′ für $-COOR_1$ steht und $R_1$ $C_1$-$C_4$-Alkyl bedeutet und n und R die unter der Formel I gegebene Bedeutung haben, kann gewünschtenfalls mit 2,4-Bis-(4-methoxyphenyl)-1,3-dithia-2,4-diphosphethan-2,4-disulfid (Lawesson's Reagens) oder einem anderen zur Einführung der Thioxogruppe geeigneten Reagens zum 3-Phenyl-oxazolidin-2-thion-5-carbonsäurederivat der Formel Id umgesetzt werden,

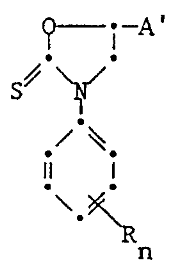

(Id)

worin A′, n und R die unter Formel Ic gegebene Bedeutung haben.

Geeignete Reagentien zur Einführung der Thioxogruppe sind z.B. Phosphorpentasulfid sowie die daraus hergeleiteteten Arylphosphinsulfide. Solche Reagentien sind z.B. in Tetrahedron 41 , 5061-5087 (1985) beschrieben.

Reaktionen zur Herstellung von Verbindungen der Formel V sind bekannt und z.B. in Tetrahedron Letters 45 (1989) 1323-1326, Chimie Therapeutique 1973 , 324 - 327 oder der FR-A 2,458,547 beschrieben.

Die Oxydation des 3-Phenyl-5-hydroxymethyl-oxazolidin-2-ons der Formel V zur 3-Phenyl-oxazolidin-2-on-5-carbonsäure der Formel Ia erfolgt z.B. gemäss Jones (Fieser & Fieser, "Reagents for Organic Synthesis", John Wiley & Sons, N.Y. Vol. I, 1967, p. 142ff.) mittels Chromschwefelsäure in einem aprotischen organischen Lösungsmittel bei einer tiefen Temperatur zwischen -10° bis Raumtemperatur. Als Lösungsmittel eignen sich Ketone wie Aceton, Methylethylketon, Acetophenon, Cyclohexanon, Dioxan oder höhere Aether wie Tetrahydrofuran oder Dipropyläther.

Die optisch aktiven Isomere der Formel I, dargestellt durch die Unterformeln (R)-I und (S)-I, bilden ebenfalls einen Gegenstand der vorliegenden Erfindung. Sie können auf an sich bekannte Weise hergestellt werden, indem man den vorangehend beschriebenen Reaktionsweg mit kommerziell erhältlichem, optisch aktivem Glycidol ((+)-III oder (-)-III) durchführt oder eine racemische 3-Phenyl-oxazolidin-2-on-5-carbonsäure der Formel Ia

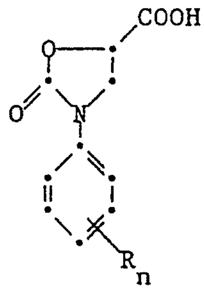

(Ia)

worin n und R die unter der Formel I gegebene Bedeutung haben, durch die an sich bekannte Methode der fraktionierten Kristallisation in Gegenwart eines chiralen Hilfsstoffes wie beispielsweise einer optisch aktiven Base inbesondere eines optisch aktiven 1-Phenylethylamins in die optisch aktive 5-(R)(-)-3-phenyl-oxazolidin-2-on-5-carbonsäure der Formel (R)-Ia

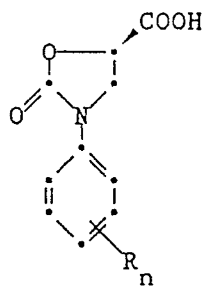

(R)-(Ia)

worin n und R die unter der Formel I gegebene Bedeutung haben und die optisch aktive 5-(S)(+)-3-Phenyl-oxazolidin-2-on-5-carbonsäure der Formel (S)-Ia auftrennt,

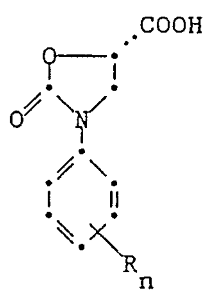

(S)-(Ia)

worin n und R die unter der Formel I gegebene Bedeutung haben, diese Isomere gegebenenfalls auf an sich bekannte Weise in die definitionsgemässen Derivate der Formel (R)-Ic und (S)-Ic

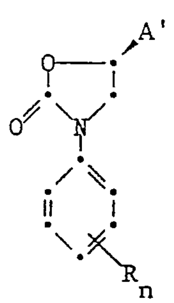

(R)-(Ic)

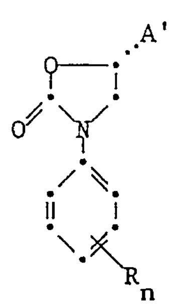

(S)-(Ic)

worin A′ für $-COOR_1$ und $R_1$ für $C_1$-$C_4$-Alkyl steht und n und R die unter der Formel II gegebene Bedeutung haben, überführt, um anschliessend durch Umsetzung mit einem zur Einführung der Thioxogruppe befähigten Reagenz die entsprechenden Verbindungen der Formeln (R)-Id und (S)-Id

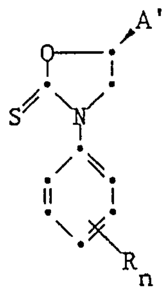 (R)-(Id) 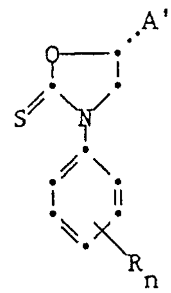 (S)-(Id)

worin A' für $-COOR_1$ und $R_1$ für $C_1$-$C_4$-Alkyl steht, und n und R die unter der Formel I gegebene Bedeutung haben, zu erhalten, welche gegebenenfalls anschliessend auf an sich bekannte Weise in die anderen definitionsgemässen Derivate der Formeln (R)-Ie und (S)-Ie

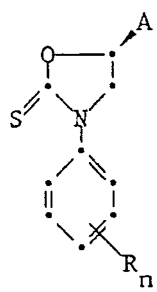 (R)-(Ie) 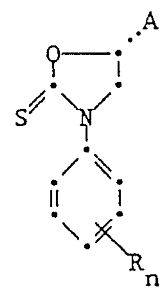 (S)-(Ie)

worin A, n und R die unter Formel I angegebene Bedeutung hat, überführt werden können.

Geeignete Reagenzien zur Einführung der Thioxogruppe sind beispielsweise Phosphorpentasulfid sowie die daraus hergeleiteten Arylthionophosphinsulfide. Derartige Reagenzien sind in Tetrahedron 41 , 5061-5087 (1985) beschrieben.

Als besonders gut geeignetes Reagenz zur Einführung der Thioxogruppe hat sich 2,4-Bis-(4-methoxyphenyl)-1,3-dithia-2,4-diphosphetan-2,4-disulfid (Lawesson's Reagenz) erwiesen.

Die Ueberführung der Verbindungen der Formeln (R)-Ia und (S)-Ia in die definitionsgemässen Derivate der Formeln (R)-Ib bzw. (S)-Ib erfolgt nach an sich bekannter Weise, z.B.:

- durch Umsetzung mit Alkoholen $R_1OH$, Mercaptanen $R_1SH$ oder Aminen $R_2R_3NH$ nach an sich bekannten Verfahren.

Die Ueberführung der racemischen 3-Phenyl-oxazolidin-2-thion-5-carbonsäuren der Formel Ie sowie deren optisch aktiven Isomeren der Formeln (R)-Ie und (S)-Ie in die anderen definitionsgemässen Derivate der Formel Ie erfolgt ebenfalls nach an sich bekannter Weise, z.B.:

- bei Säurechloriden (A = -COCl)
durch Umsetzung der freien Carbonsäuren mit geeigneten Chlorierungsmitteln, wie Thionylchlorid, Oxalylchlorid, Phosgen oder $PCl_5$.

- bei Amiden

(A = 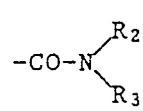 )

durch Umsetzung der Säureester, Säurechloride oder freien Carbonsäuren mit Aminen

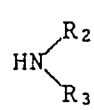

- bei Thioestern ($A = -COSR_1$)
durch Umsetzung der Säurechloride mit Mercaptanen $HSR_1$;

- bei Alkalimetall- oder Erdalkalimetallsalzen
durch Umsetzung der freien Carbonsäuren mit Alkalimetall- oder Erdalkalimetallhydroxiden, -alkoholaten

oder -carbonaten, wie Na-, K-, Li-, Ca- und Mg-hydroxid, Natrium- und Kaliummethylat und -ethylat;
- bei Aminsalzen
durch Umsetzung der freien Carbonsäure mit Aminen $N(R_2)_3$.

Die Erfindung betrifft auch pflanzenwachstumsregulierende Mittel, welche einen neuen Wirkstoff der Formel I enthalten, sowie Verfahren zur Hemmung des Pflanzenwuchses.

Pflanzenwachstumsregulatoren sind Substanzen, die in/an der Pflanze agronomisch erwünschte biochemische und/oder physiologische und/oder morphologische Veränderungen bewirken.

Die in den erfindungsgemässen Mitteln enthaltenen Wirkstoffe beeinflussen das Pflanzenwachstum je nach Applikationszeitpunkt, Dosierung, Applikationsart und Umweltbedingungen in verschiedener Weise. Pflanzenwuchsregulatoren der Formel I können zum Beispiel das vegetative Wachstum von Pflanzen hemmen. Diese Art der Wirkung ist von Interesse auf Rasenflächen, im Zierpflanzenbau, in Obstplantagen, an Strassenböschungen, auf Sport- und Industrieanlagen, aber auch bei der gezielten Hemmung von Nebentrieben wie bei Tabak. Im Ackerbau führt die Hemmung des vegetativen Wachstums bei Getreide über eine Halmverstärkung zu reduziertem Lager, ähnliche agronomische Wirkung erreicht man in Raps, Sonnenblumen, Mais und anderen Kulturpflanzen. Des weiteren kann durch Hemmung des vegetativen Wachstums die Anzahl Pflanzen pro Fläche erhöht werden. Ein weiteres Einsatzgebiet von Wuchshemmern ist die selektive Kontrolle von bodendeckenden Pflanzen in Plantagen oder weitreihigen Kulturen durch starke Wuchshemmung, ohne diese Bodendecker abzutöten, sodass die Konkurrenz gegenüber der Hauptkultur ausgeschaltet ist, die agronomisch positiven Effekte wie Erosionsverhinderung, Stickstoffbindung und Bodenlockerung aber erhalten bleiben.

Unter einem Verfahren zur Hemmung des Pflanzenwachstums ist eine Steuerung der natürlichen Pflanzenentwicklung zu verstehen, ohne den von genetischen Eigenschaften determinierten Lebenszyklus der Pflanze im Sinne einer Mutation zu verändern. Das Verfahren der Wuchsregulierung wird zu einem im Einzelfall zu bestimmenden Entwicklungszeitpunkt der Pflanze angewendet. Die Applikation der Wirkstoffe der Formel I kann vor oder nach dem Auflaufen der Pflanzen erfolgen, beispielsweise bereits auf die Samen oder die Sämlinge, auf Wurzeln, Knollen, Stengel, Blätter, Blüten oder andere Pflanzenteile. Dies kann z.B. durch Aufbringen des Wirkstoffes selbst oder in Form eines Mittels auf die Pflanzen und/oder durch Behandlung des Nährmediums der Pflanze (Erdboden) geschehen.

Bevorzugt setzt man die erfindungsgemässen Verbindungen der Formel I zur Wachstumshemmung in dikotylen Kulturen mit postemergenter Applikation ein.

Die Verbindungen der Formel I werden in unveränderter Form oder vorzugsweise als Mittel zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen, in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder -ethylether, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäure-methyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfate oder -sulfonate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2-Sulfonsäuregruppen und einen Fettsäurerest mit 8 bis 22 C-Atomen. Alkylarylsulfonate sind z.B. die Na-, Ca- oder Triethanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes oder Phospholipide in Frage.

Als nicht ionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 10 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykolethergruppen und 10 bis 100 Propylenglykolethergruppen enthaltenden Polyethylenoxidaddukte an Polypropylenglykol, Aethylendiaminopolypropylenglykol und Alkylpolypropylenglykol mit 1 bis 10 Kohlenstoffatomen in der Alkylkette, die genannten Verbindungen enthalten üblicherweise pro Propylenglykol-Einheit 1 bis 5 Ethylenglykoleinheiten.

Als Beispiele nichtionischer Tenside seien Nonylphenolpolyethoxyethanole, Ricinusölpolyethylenglykolether, Polypropylen-polyethylenoxidaddukte, Tributylphenoxypolyethoxyethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyldi(2-chlorethyl)-ethylammoniumbromid.

Die in der Formulierungstechnik gebräuchlichen Tenside sind u.a. in folgenden Publikationen beschrieben:

"Mc Cutcheon's Detergents and Emulsifiers Annual" MC Publishing Corp., Ridgewood, New Jersey, 1981;

H. Stache, "Tensid-Taschenbuch", 2. Aufl., C. Hanser Verlag, München, Wien, 1981;

M. and J. Ash. "Encyclopedia of Surfactants", Vol. I-III, Chemical Publishing Co., New York, 1980-1981.

Die agrochemischen Zubereitungen enthalten in der Regel 0,1 bis 95 %, insbesondere 0,1 bis 80 % Wirkstoff der Formel I, 1 bis 99,9 % eines festen oder flüssigen Zusatzstoffes und 0 bis 25 %, insbesondere 0,1 bis 25 % eines Tensides.

Insbesondere setzen sich bevorzugte Formulierungen folgendermassen zusammen: (% = Gewichtsprozent).

Emulgierbare Konzentrate :

Aktiver Wirkstoff: 1 bis 20 %, bevorzugt 5 bis 10 %
oberflächenaktive Mittel: 5 bis 30 %, vorzugsweise 10 bis 20 %
flüssiges Trägermittel: 50 bis 94 %, vorzugsweise 70 bis 85 %

Stäubemittel :

Aktiver Wirkstoff: 0,1 bis 10 %, vorzugsweise 0,1 bis 1 %
festes Trägermittel: 99,9 bis 90 %, vorzugsweise 99,9 bis 99 %

Suspensions-Konzentrate :

Aktiver Wirkstoff: 5 bis 75 %, vorzugsweise 10 bis 50 %
Wasser: 94 bis 24 %, vorzugsweise 88 bis 30 %

oberflächenaktives Mittel: 1 bis 40 %, vorzugsweise 2 bis 30 %

Benetzbares Pulver :

Aktiver Wirkstoff: 0,5 bis 90 %, vorzugsweise 1 bis 80 %

oberflächenaktives Mittel: 0,5 bis 20 %, vorzugsweise 1 bis 15 %

festes Trägermittel: 5 bis 99 %, vorzugsweise 15 bis 90 %

Granulate :

Aktiver Wirkstoff: 0,5 bis 30 %, vorzugsweise 3 bis 15 %

festes Trägermittel: 99,5 bis 70 %, vorzugsweise 97 bis 85 %.

Während als Handelsware eher konzentrierte Mittel bevorzugt werden, verwendet der Endverbraucher in der Regel verdünnte Mittel. Die Anwendungsformen können bis hinab zu 0,001 % an Wirkstoff verdünnt werden. Die Aufwandmengen betragen in der Regel 0,01 bis 10 kg AS/ha, vorzugsweise 0,025 bis 5 kg AS/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

## Herstellungsbeispiele

Die folgenden Beispiele veranschaulichen die Herstellung einiger erfindungsgemässer Verbindungen.

### Beispiel 1: Herstellung von racemischem 3-(3,5-Bis-trifluormethylphenyl)-5-carboxyl-oxazolidin-2-on

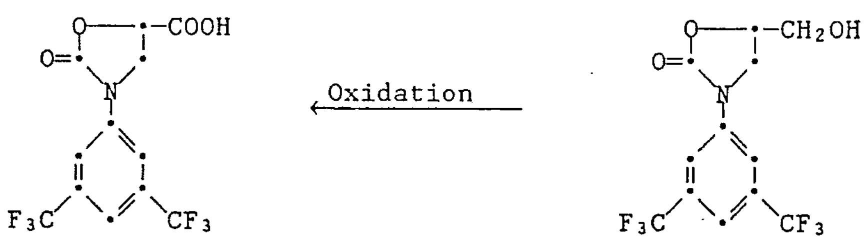


Zu einer auf 0° C gekühlten Lösung von 24,7 g (0.075 Mol) 3-(3,5-Bis-trifluormethylphenyl)-5-hydroxymethyl-oxazolidin-2-on (hergestellt nach Chimie Thérapeutique 1973 , 324 - 327, aus 3,5-Bis-trifluormethylanilin und rac. Glycidol) in 500 ml Aceton tropft man unter Rühren 62 ml Chromschwefelsäure zu (3.25 molar $CrO_3$ und 5.29 molar $H_2SO_4$). Nachdem alles zugegeben ist, lässt man 14 Stunden bei Raumtemperatur weiterrühren. Dann gibt man zur entstandenen grünen Suspension zuerst 100 ml Isopropanol, dann 1 l Aethyläther und zuletzt 1 l Salzsole. Nachdem sich die Chromsalze gelöst haben, trennt man die organische Phase ab und extrahiert die wässrige Phase mit Aether. Die organischen Phasen werden über Magnesiumsulfat getrocknet, und am Rotationsverdampfer eingeengt. Der Rückstand ergibt 25,86 g beige kristalline Substanz, welche aus Ether/Petrolether umkristallisiert, 19,42 g Titelprodukt in Form weisser Kristalle vom Schmelzpunkt 165 - 169° C ergibt.

### Beispiel 2: Herstellung von racemischem 3-(3,5-Bis-trifluormethylphenyl)-5-methoxycarbonyl-oxazolidin-2-on

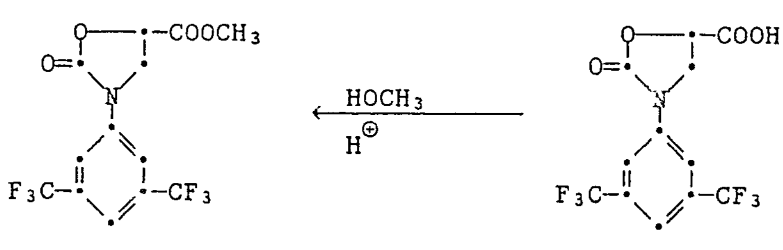


Ein Gemisch von 52,2 g (0,152 Mol) 3-(3,5-Bistrifluormethylphenyl)-5-carboxyl-oxazolidin-2-on (Beispiel 1) und 77,8 g ®Amberlyst-Harz (20 - 50 mesh, stark sauer) in 600 ml absolutem Methanol wird während 2

Stunden am Rückfluss erhitzt und über Nacht stehen gelassen. Dann wird das Harz abgenutscht und das Filtrat am Rotationsverdampfer eingeengt. Der braune, ölige Rückstand wird in Ethylether aufgenommen, mit Natriumbicarbonat und dann mit Wasser neutral gewaschen, über Magnesiumsulfat getrocknet und zur Trockne eingeengt.

Man isoliert 50,9 g rohes 3-(3,5-Bistrifluormethylphenyl)-5-methoxycarbonyl-oxazolidin-2-on als oranges Oel. Dieses wird in Methylenchlorid gelöst und mit Silicumoxid verrührt. Nach Abnutschen der Kieselsäure wird die Lösung zur Trockne eingedampft und der Rückstand in Petroläther gut verrührt. Die entstandenen Kristalle werden abfiltriert und im Vakuum bei 40° C getrocknet. Man erhält so 45,9 g (84.5 % der Theorie) Methylester in Form weisser Kristalle, die bei 71 - 74° C schmelzen.

In analoger Weise werden die Verbindungen der Tabelle 1 hergestellt.

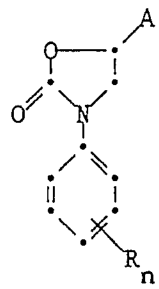

(Ib)

Tabelle 1:

| Verbindung Nr. | A | $R_n$ | phys. Daten |
|---|---|---|---|
| 1.01 | (R)-$COOCH_3$ | 3,5 $(CF_3)_2$ | Smp. 86° |
| 1.02 | ± $-COOCH_3$ | 3,5 $(CF_3)_2$ | Smp. 71 - 74° |
| 1.03 | (S)-$COOCH_3$ | 3,5 $(CF_3)_2$ | |
| 1.04 | (R)-COOH | 3,5 $(CF_3)_2$ | |
| 1.05 | (S)-COOH | 3,5 $(CF_3)_2$ | |
| 1.06 | ± -COOH | 3,5 $(CF_3)_2$ | Smp. 165 - 169° |
| 1.07 | (R)-$COOCH_2CH_3$ | 3,5 $Cl_2$ | |
| 1.08 | (S)-$COOCH_2CH_3$ | 3,5 $Cl_2$ | |
| 1.09 | ± $-COOCH_2CH_3$ | 3,5 $Cl_2$ | |
| 1.10 | (R)-$COO-CH(CH_3)-CH_3$ | 3,5 $F_2$ | |
| 1.11 | (S)-$COO-CH(CH_3)-CH_3$ | 3,5 $F_2$ | |
| 1.12 | ± $-COO-CH(CH_3)-CH_3$ | 3,5 $F_2$ | |
| 1.13 | (R)-$COO-CH_2CH_2CH_2CH_3$ | 3,5 $Br_2$ | |
| 1.14 | (S)-$COO-CH_2CH_2CH_2CH_3$ | 3,5 $Br_2$ | |
| 1.15 | ± $-COO-CH_2CH_2CH_2CH_3$ | 3,5 $Br_2$ | |
| 1.16 | (R)-COO-tert.-$C_4H_9$ | 3,5 $(CF_3)_2$ | |
| 1.17 | (S)-COO-tert.-$C_4H_9$ | 3,5 $(CF_3)_2$ | |
| 1.18 | ±-COO-tert.-$C_4H_9$ | 3,5 $(CF_3)_2$ | Smp. 137 - 139° |
| 1.19 | (R)-$COOCH_2-CH=CH_2$ | 3-Cl | |
| 1.20 | (S)-$COOCH_2-CH=CH_2$ | 3-Cl | |
| 1.21 | ±-$COOCH_2-CH=CH_2$ | 3-Cl | |
| 1.22 | (R)-$COOCH_2-C\equiv CH$ | 3,5 $(CF_3)_2$ | |

Tabelle 1: (Fortsetzung)

| Verbindung Nr. | A | $R_n$ | phys. Daten |
|---|---|---|---|
| 1.23 | (S)-$COOCH_2-C\equiv CH$ | 3,5 $(CF_3)_2$ | |
| 1.24 | ±-$COOCH_2-C\equiv CH$ | 3,5 $(CF_3)_2$ | Oel |
| 1.25 | (R)-$COOCH_2-CH=CH(CH_3)$ | 3,5 $I_2$ | |
| 1.26 | (S)-$COOCH_2-CH=CH(CH_3)$ | 3,5 $I_2$ | |
| 1.27 | ±-$COOCH_2-CH=CH(CH_3)$ | 3,5 $I_2$ | |
| 1.28 | (R)-$COOCH_2-C\equiv C-CH_3$ | 3-F | |
| 1.29 | (S)-$COOCH_2-C\equiv C-CH_3$ | 3-F | |
| 1.30 | ±-$COOCH_2-C\equiv C-CH_3$ | 3-F | |
| 1.31 | (R)-COCl | 3,5 $(CF_3)_2$ | |
| 1.32 | (S)-COCl | 3,5 $(CF_3)_2$ | |
| 1.33 | ± -COCl | 3,5 $(CF_3)_2$ | |
| 1.34 | (R)-$COSCH_3$ | 3,5 $(CF_3)_2$ | |
| 1.35 | (S)-$COSCH_3$ | 3,5 $(CF_3)_2$ | |
| 1.36 | ± -$COSCH_3$ | 3,5 $(CF_3)_2$ | Smp. 54 - 56° |
| 1.37 | (R)-COS-n-$C_4H_9$ | 2, 3, 5 $F_3$ | |
| 1.38 | (S)-COS-n-$C_4H_9$ | 2, 3, 5 $F_3$ | |
| 1.39 | ± -COS-n-$C_4H_9$ | 2, 3, 5 $F_3$ | |
| 1.40 | (R)-$CON(CH_3)(CH_3)$ | 3, 5 $Cl_2$ | |
| 1.41 | (S)-$CON(CH_3)(CH_3)$ | 3, 5 $Cl_2$ | |
| 1.42 | ± -$CON(CH_3)(CH_3)$ | 3, 5 $Cl_2$ | |
| 1.43 | (R)-$COO^{\ominus}Na^{\oplus}$ | 3, 5 $(CF_3)_2$ | |
| 1.44 | (S)-$COO^{\ominus}Na^{\oplus}$ | 3, 5 $(CF_3)_2$ | |
| 1.45 | ± -$COO^{\ominus}Na^{\oplus}$ | 3, 5 $(CF_3)_2$ | Smp. 105° zers. |
| 1.46 | $[(R)-COO^{\ominus}]^{2\ominus}Ca^{2\oplus}$ | 3, 5 $F_2$ | |
| 1.47 | $[(S)-COO^{\ominus}]^{2\ominus}Ca^{2\oplus}$ | 3, 5 $F_2$ | |

Tabelle 1: (Fortsetzung)

| Verbindung Nr. | A | $R_n$ | phys. Daten |
|---|---|---|---|
| 1.48 | $[\pm\ -COO^{\ominus}]^{2\ominus}Ca^{2\oplus}$ | 3, 5 $F_2$ | |
| 1.49 | (R)-$COO^{\ominus}\overset{\oplus}{N}H(C_4H_9)_3$ | 3, 5 $Cl_2$ | |
| 1.50 | (S)-$COO^{\ominus}\overset{\oplus}{N}H(C_4H_9)_3$ | 3, 5 $Cl_2$ | |
| 1.51 | ± $-COO^{\ominus}\overset{\oplus}{N}H(C_4H_9)_3$ | 3, 5 $Cl_2$ | |
| 1.52 | (R)-$COO^{\ominus}\overset{\oplus}{N}H_4$ | 3, 5 $(CCl_3)_2$ | |
| 1.53 | (S)-$COO^{\ominus}\overset{\oplus}{N}H_4$ | 3, 5 $(CCl_3)_2$ | |
| 1.54 | ± $-COO^{\ominus}\overset{\oplus}{N}H_4$ | 3, 5 $(CCl_3)_2$ | |
| 1.55 | (R) 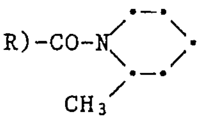 | 3-Br | |
| 1.56 | (S) 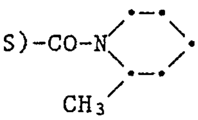 | 3-Br | |
| 1.57 | ± 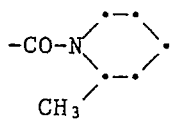 | 3-Br | |
| 1.58 | (R)-CO-NH- 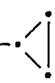 | 4-$CF_3$ | |
| 1.59 | (S)-CO-NH- 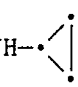 | 4-$CF_3$ | |
| 1.60 | ± -CO-NH- 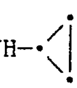 | 4-$CF_3$ | |
| 1.61 | ± $-COS-n-C_4H_9$ | 3, 5 $(CF_3)_2$ | Oel |
| 1.62 | ± $-COOCH_2CH=CH_2$ | 3, 5 $(CF_3)_2$ | Oel |
| 1.63 | ± $-COO^{\ominus}H\overset{\oplus}{N}(n-C_4H_9)_3$ | 3, 5 $(CF_3)_2$ | Smp. 65 - 72° |
| 1.64 | ± -CONH- 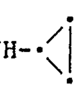 | 3, 5 $(CF_3)_2$ | Smp. 132 - 134° |

Tabelle 1: (Fortsetzung)

| Verbindung Nr. | A | $R_n$ | phys. Daten |
|---|---|---|---|
| 1.65 | ± 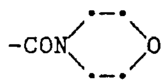 | 3, 5 $(CF_3)_2$ | Smp. 150 - 151° |
| 1.66 | ± 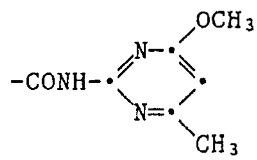 | 3, 5 $(CF_3)_2$ | Smp. 50 - 52° |
| 1.67 | ± $-COOH$ | 3, 5 $Cl_2$ | Smp. 158-160° |
| 1.68 | ± $-COOCH_2C{\equiv}CH$ | 3, 5 $Cl_2$ | Smp. 149-152° |
| 1.69 | ± $-COOCH_2CH{=}CH_2$ | 3, 5 $Cl_2$ | Smp. 115-117° |

Formulierungsbeispiele:

Beispiel F 1: Formulierungsbeispiele für Wirkstoffe der Formel I (% = Gewichtsprozent)

| a) Spritzpulver | a) | b) | c) |
|---|---|---|---|
| Wirkstoff aus Tabelle 1 | 20 % | 50 % | 0,5 % |
| Na-Ligninsulfonat | 5 % | 5 % | 5 % |
| Na-Laurylsulfat | 3 % | - | - |
| Na-Diisobutylnaphthalinsulfonat | - | 6 % | 6 % |
| Octylphenolpolyethylenglykoläther /7-8 Mol AeO) | - | 2 % | 2 % |
| Hochdisperse Kieselsäure | 5 % | 27 % | 27 % |
| Kaolin | 67 % | 10 % | - |
| Natriumchlorid | - | - | 59,5 % |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| b) Emulsions-Konzentrat | a) | b) |
|---|---|---|
| Wirkstoff aus Tabelle 1 | 10 % | 1 % |
| Octylphenolpolyethylenglykolether (4-5 Mol AeO) | 3 % | 3 % |
| Ca-Dodecylbenzolsulfonat | 3 % | 3 % |
| Ricinusölpolyglykolether (36 Mol AeO) | 4 % | 4 % |
| Cyclohexanon | 30 % | 10 % |
| Xylolgemisch | 50 % | 79 % |

Aus diesem Konzentrat können durch Verdünnen mit Wasser Emulsionen jeder gewünschten Konzentration hergestellt werden.

| c) Stäubemittel | a) | b) |
|---|---|---|
| Wirkstoff aus Tabelle 1 | 0,1 % | 1 % |
| Talkum | 99,9 % | - |
| Kaolin | - | 99 % |

Man erhält anwendungsfertige Stäubemittel, indem der Wirkstoff mit dem Träger vermischt und auf einer geeigneten Mühle vermahlen wird.

| d) Extruder Granulat | a) | b) |
|---|---|---|
| Wirkstoff aus Tabelle 1 | 10 % | 1 % |
| Na-Ligninsulfonat | 2 % | 2 % |
| Carboxymethylcellulose | 1 % | 1 % |
| Kaolin | 87 % | 96 % |

Der Wirkstoff wird mit den Zusatzstoffen vermischt, vermahlen und mit Wasser angefeuchtet. Dieses Gemisch wird extrudiert und anschliessend im Luftstrom getrocknet.

| e) Umhüllungs-Granulat | |
|---|---|
| Wirkstoff aus Tabelle 1 | 3 % |
| Polyethylenglykol (MG 200) | 3 % |
| Kaolin | 94 % |

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

| f) Suspensions-Konzentrat | a) | b) |
|---|---|---|
| Wirkstoff aus Tabelle 1 | 40 % | 5 % |
| Ethylenglykol | 10 % | 10 % |
| Nonylphenolpolyethylenglykolether (15 Mol AeO) | 6 % | 1 % |
| Na-Ligninsulfonat | 10 % | 5 % |
| Carboxymethylcellulose | 1 % | 1 % |
| 37 %ige wässrige Formaldehyd-Lösung | 0,2 % | 0,2 % |
| Silikonöl in Form einer 75 %igen wässrigen Emulsion | 0,8 % | 0,8 % |
| Wasser | 32 % | 77 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

| g) Salzlösung | |
|---|---|
| Wirkstoff aus Tabelle 1 | 5 % |
| Isopropylamin | 1 % |
| Octylphenolpolyethylenglykolether (78 Mol AeO) | 3 % |
| Wasser | 91 % |

Biologische Beispiele:

Die folgenden Beispiele veranschaulichen die Wirkung einiger erfindungsgemässer Verbindungen in pflanzenwuchsregulierenden Versuchsanordnungen.

Beispiel B1: Wuchshemmung bei tropischen Bodendecker-Leguminosen (Cover-Crops)

Die Pflanzen (z.B. Centrosema pubescens oder Psophocarpus palustris) werden in 4 cm - Torftöpfen mit Landerde (45 %), Torf (45 %) und Zonolit (10 %) durch Stecklinge vermehrt. Die Anzucht erfolgt im Gewächshaus bei einer Tagestemperatur von 27° C und einer Nachttemperatur von 23° C. Die Beleuchtungsdauer ist mindestens 14 Stunden/Tag bei einer Intensität von mindestens 7000 Lux.

Ca. 50 Tage nach Ansatz der Stecklinge erfolgt das Vertopfen in 13 cm-Töpfe, 4 - 5 Pflanzen/Topf. Nach weiteren 60 Tagen werden die Pflanzen auf ca. 15 cm Höhe zurückgeschnitten und appliziert. Dabei werden sie mit 0.3 bis 3 kg Wirkstoff/ha (in der Regel 25 %ig formuliert) in wässriger Spritzbrühe besprüht. Die Wasseraufwandmenge beträgt ca. 200 l/ha.

4 Wochen nach der Applikation wird der Versuch ausgewertet. Es werden dabei der Neuzuwachs im Vergleich zur Kontrolle bonitiert und gewogen und die Phytotoxizität bewertet. In diesem Versuch zeigen die mit den Wirkstoffen aus der Tabelle 1 behandelten Pflanzen eine deutliche Reduktion des Neuzuwachses ohne dass dabei die Versuchspflanzen geschädigt werden.

Beispiel B2: Wuchsregulierung bei Sojabohnen

Die Pflanzen (z.B. der Sorte Williams) werden in 11 cm - Tontöpfen mit Landerde (45 %), Torf (45 %) und Zonolit (10 %) angesät und in der Klimakammer bei einer Tagestemperatur von 24° C und einer Nachttemperatur von 19° C angezogen. Die Beleuchtungsdauer ist 16 Stunden pro Tag bei einer Intensität von ca. 2900 Lux.

Ca. 24 Tage nach der Saat erfolgt das Umtopfen in 18 cm - Töpfe, 2 Pflanzen/Topf. Nach weiteren 12 Tagen und im Stadium von 5 - 6 trifol. Blätter findet die Applikation mit bis zu 100 g Wirkstoff/ha statt, in der Regel 25 %ig formuliert und in wässriger Spritzbrühe. Die Wasseraufwandmenge beträgt ca. 200 l/ha.

Ca. 4 Wochen nach der Applikation findet die Auswertung statt. Im Vergleich zu unbehandelten Kontrollpflanzen bewirken die erfindungsgemässen Wirkstoffe aus der Tabelle 1 eine merkliche Wuchshemmung ohne Phytotoxizität.

Beispiel B3: Selektive Wuchshemmung von Raps und Klee in Mais

Die Verbindungen der Tabelle 1 sind sehr gut dazu geeignet, bodendeckende Pflanzen in einer Maiskultur selektiv stark zu hemmen und so die Wasser-und Nährstoffkonkurrenz der Untersaat auszuschalten bei gleichzeitiger Aufrechterhaltung der agronomisch erwünschten Wirkungen der Untersaat wie Erosionsschutz, Stickstoffbindung und Reduzierung der Bodenverdichtung. Dies wird für die Kulturkombinationen Mais/Weissklee und Mais/Raps wie folgt geprüft:

Mais 'Blizzard' wird in 15 cm-Töpfen mit Landerde ausgesät und im Gewächshaus bei Tag-/Nachttemperaturen von 22/19° C und einer Beleuchtungsdauer von mindestens 13.5 Stunden/Tag für die postemergente Applikation 15 Tage kultiviert, die preemergente Applikation erfolgt einen Tag nach der Saat.

Raps 'Bienvenue' wird in 15 cm-Töpfen mit Landerde ausgesät, 7 Tage bei Tag-/Nachttemperaturen von 22/19° C, dann 17 Tage bei 10/5° C, dann 7 Tage bei 15/10° C bis zur Applikation kultiviert.

Weissklee 'Ladino' wird in 15 cm-Töpfen mit einer Mischung aus Landerde (60 %), Torfsubstrat (30) und Zonolite (10 %) 40 Tage bei Tag-/Nachttemperaturen von 21/18° C und einer Mindestbeleuchtungsdauer von 13.5 Stunden angezogen.

Düngung und Bewässerung erfolgt in allen Kulturen nach Bedarf, die Applikation der Verbindung Nr. 1.01 (25%ig formuliert) erfolgt in 200 l Wasser/ha und Aufwandmengen von 30 bis 500 g/ha. 28 Tage nach Applikation wird die Höhe des Neuzuwachses gemessen und die Wirkung in Prozent Wuchshemmung im Vergleich zur unbehandelten Kontrolle dargestellt. 100 % Wirkung bedeutet völliger Wachstumsstopp, 0 % Wirkung bedeutet Wachstum wie unbehandelte Kontrolle.

Die geprüften Verbindungen der Tabelle 1 zeigten in diesem Versuch gute Wuchshemmung beim Klee und beim Raps, während der Mais im Wachstum nicht gehemmt wurde.

Beispiel B4: Selektive Wuchshemmung von Unkräutern in Kulturpflanzen

Die Verbindungen der Tabelle 1 sind auch dazu geeignet, in Kulturpflanzenbeständen selektiv Unkräuter stark zu hemmen und so deren Konkurrenz auszuschalten ohne sie völlig abzutöten. Dies wird mit den Kulturpflanzen Gerste, Weizen und Mais sowie mit den Unkräutern Ipomoea purpurea, Solanum nigrum, Xanthium strumarium, Chenopodium album, Galium aparine, Veronica persica und Chrysanthemum leucanthemum folgendermassen geprüft:

Die genannten Kulturpflanzen und Unkräuter werden im Gewächshaus angezogen und im 4-6-Blattstadium mit einer wässrigen Wirkstoffsuspension der Verbindungen der Tabelle 1 besprüht. Die behandelten Pflanzen werden im Gewächshaus weiterkultiviert. 15 Tage nach Behandlung wird ausgewertet und die Wuchshemmung in Prozent angegeben (Tabelle 2). 0 = keine Wuchshemmung, 100 = völliger Wuchsstopp nach Behandlung. Als Beispiel sind in der Tabelle die Resultate für Verbindungs-Nr. 1.02 (Tabelle 1) angegeben.

Tabelle 2:

| Pflanzen | Wuchshemmung in % bei (g Wirkstoff/ha) | | | | |
|---|---|---|---|---|---|
| | 60 | 125 | 250 | 500 | 1000 |
| Gerste | 0 | 0 | 0 | 0 | 10 |
| Weizen | 0 | 0 | 0 | 0 | 0 |
| Mais | 0 | 0 | 0 | 0 | 0 |
| Jpomoea | 10 | 60 | 70 | 90 | 90 |
| Solanum | 0 | 40 | 70 | 80 | 80 |
| Xanthium | 10 | 40 | 80 | 80 | 90 |
| Chenopodium | 0 | 20 | 30 | 70 | 70 |
| Galium | 10 | 20 | 70 | 90 | 100 |
| Chrysanthemum | 40 | 60 | 80 | 90 | 90 |
| Veronica | 30 | 50 | 60 | 80 | 90 |

## Ansprüche

1. 3-Phenyl-oxazolidin-2-on- und -2-thion-5-carbonsäurederivate in allen stereoisomeren Formen der Formel I

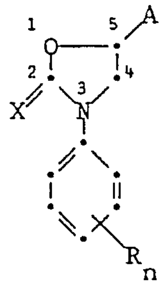

(I)

worin X für Sauerstoff oder Schwefel;
A für $-COOR_1$, $-COSR_1$, $-COO^{\ominus}M^{\oplus}$, $-CONR_2R_3$ oder $-COCl$;
R für Wasserstoff, Halogen, Mono-, Di- oder Trihalomethyl;

n für 1, 2 oder 3;
$R_1$ für Wasserstoff, $C_1$-$C_4$-Alkyl, $C_2$-$C_6$-Alkenyl oder $C_2$-$C_6$-Alkinyl;
$R_2$ und $R_3$ unabhängig voneinander für Wasserstoff, $C_1$-$C_4$-Alkyl, $C_2$-$C_6$-Alkenyl, $C_2$-$C_6$-Alkinyl, $C_3$-$C_7$-Cycloalkyl, Aryl oder Heterocyclyl; oder $R_2$ und $R_3$ zusammen mit dem Stickstoffatom, an welches sie gebunden sind, für einen gegebenenfalls bis zu dreifach durch $C_1$-$C_4$-Alkyl oder $C_1$-$C_4$-Alkoxy substituierten gesättigten 3- bis 7-gliedrigen Heterocyclus, der noch ein weiteres Heteroatom ausgewählt aus der Gruppe O, N und S enthalten kann, stehen; und $M^{\oplus}$ das Aequivalent eines Alkali- oder Erdalkalimetallkations oder $HN^{\oplus}(R_2)_3$ bedeutet.

2. Optisch aktive Verbindungen gemäss Anspruch 1 der Formeln (R)-I und (S)-I

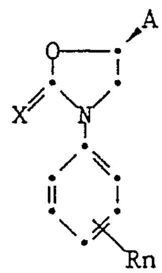
(R)-(I)

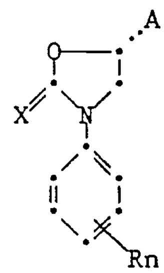
(S)-(I)

worin A, n, R und X die im Anspruch 1 gegebene Bedeutung haben.

3. Verbindungen der Formel I gemäss Anspruch 1, bei denen A für -$COOR_1$ oder -$COO^{\ominus}M^{\oplus}$, R für Halogen oder Trifluoromethyl und n für 1 oder 2 steht und $R^1$ und $M^{\oplus}$ die im Anspruch 1 gegebene Bedeutung haben.

4. 3-Phenyl-oxazolidin-2-on-5-carbonsäurederivate gemäss Anspruch 1 in allen stereoisomeren Formen der Formel Ib

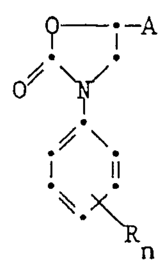
(Ib)

worin A, n und R die im Anspruch 1 gegebene Bedeutung haben.

5. 3-Phenyl-oxazolidin-2-thion-5-carbonsäurederivate gemäss Anspruch 1 in allen stereoisomeren Formen der Formel Ie

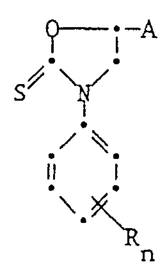
(Ie)

worin A, n und R die im Anspruch 1 gegebene Bedeutung haben.

6. Ein 3-Phenyl-oxazolidin-2-on gemäss Anspruch 1 ausgewählt aus der Gruppe bestehend aus
3-(3,5-Bis-trifluormethylphenyl)-oxazolidin-2-on-5-carbonsäure,
3-(3,5-Bis-trifluormethylphenyl)-oxazolidin-2-on-5-carbonsäuremethylester,
5-(R)-3-(3,5-Bis-trifluormethylphenyl)-oxazolidin-2-on-5-carbonsäure,
5-(R)-3-(3,5-Bis-trifluormethylphenyl)-oxazolidin-2-on-5-carbonsäuremethylester,
5-(S)-3-(3,5-Bis-trifluormethylphenyl)-oxazolidin-2-on-5-carbonsäure,
5-(S)-3-(3,5-Bis-trifluormethylphenyl)-oxazolidin-2-on-5-carbonsäuremethylester,

5-(3,5-Bis-trifluormethylphenyl)-oxazolidin-2-on-5-carbonsäuretributylammoniumsalz.

7. Verfahren zur Herstellung eines 3-Phenyl-oxazolidin-2-ons und eines 3-Phenyl-oxazolidin-2-thions der Formel I gemäss Anspruch 1, dadurch gekennzeichnet, dass man ein Anilin der Formel II

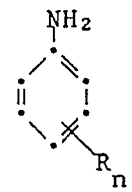

(II)

worin n und R die im Anspruch 1 gegebene Bedeutung haben, mit racemischem Glycidol ((±)-2,3-Epoxy-1-propanol) der Formel III

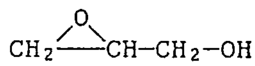

(III)

zum 1,2-Propandiolanilid der Formel IV umsetzt,

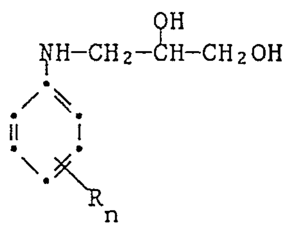

(IV)

worin n und R die im Anspruch 1 gegebene Bedeutung haben, dieses Diol dann in einem geeigneten Lösungsmittel, in Gegenwart einer Base mit Diethylcarbonat zum 3-Phenyl-5-hydroxymethyloxazolidin-2-on der Formel V cyclisiert,

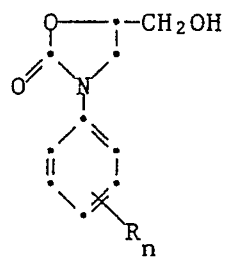

(V)

worin n und R die im Anspruch 1 gegebene Bedeutung haben und diese Verbindung dann mit einem oxidierenden Mittel zur 3-Phenyl-oxazolidin-2-on-5-carbonsäure der Formel Ia oxidiert,

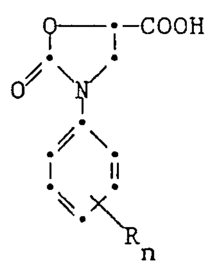

(Ia)

worin n und R die im Anspruch 1 gegebene Bedeutung haben, und diese Verbindung gewünschtenfalls in an sich bekannter Weise zum Salz oder durch Umsetzen mit einer Verbindung der Formel VI

H-B (VI)
worin B das Radikal eines Alkohols, Mercaptans oder Amins bedeutet, zum Ester, Thioester oder Amid der Formel Ib umwandelt,

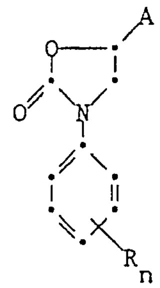

(Ib)

worin A, n und R die im Anspruch 1 gegebene Bedeutung haben.

8. Verfahren zur Herstellung von 3-Phenyl-oxazolidin-2-thion-5-carbonsäurederivaten gemäss Anspruch 1 der Formel Id

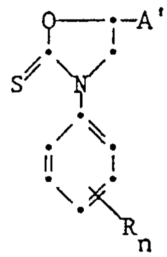

(Id)

worin n und R die in Anspruch 1 gegebene Bedeutung haben und A′ für $-COOR_1$ steht und $R_1$ $C_1$-$C_4$-Alkyl bedeutet, dadurch gekennzeichnet, dass man ein 3-Phenyl-oxazolidin-2-on-5-carbonsäurederivat der Formel Ic

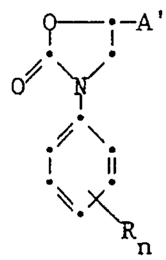

(Ic)

worin A′, n und R die oben gegebene Bedeutung haben, mit 2,4-Bis-(4-methoxyphenyl)-1,3-dithia-2,4-diphosphethan-2,4-disulfid (Lawesson's Reagens) oder einem anderen zur Einführung der Thioxogruppe geeigneten Reagens umsetzt.

9. Verfahren zur Herstellung von 3-Phenyl-oxazolidin-2-thion-5-carbonsäurederivaten gemäss Anspruch 1 der Formel Ie

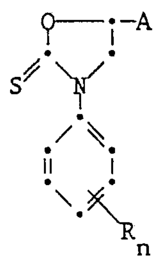

(Ie)

worin A, n und R die im Anspruch 1 gegebene Bedeutung haben, dadurch gekennzeichnet, dass man ein 3-Phenyl-oxazolidin-2-thion-5-carbonsäurederivat der Formel Id

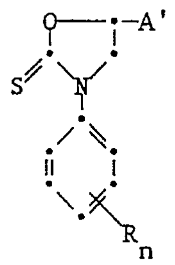

(Id)

worin n und R die im Anspruch 1 gegebene Bedeutung haben und A′ für -COOR$_1$ steht und R$_1$ -C$_1$-C$_4$ Alkyl bedeutet, in bekannter Art und Weise zu freien Säuren umwandelt, und die Säuren dann in an sich bekannter Weise zum Salz oder durch Umsetzen mit einer Verbindung der Formel VI

H-B (VI)

worin B das Radikal eines Alkohols, Merkaptans oder Amins bedeutet, zum Ester, Thioester oder Amid der Formel Ie umwandelt.

10. Ein den Pflanzenwuchs regulierendes Mittel, dadurch gekennzeichnet, dass es neben Träger- und/oder anderen Zuschlagstoffen als Wirkstoff ein 3-Phenyl-oxazolin-2-on- oder -2-thion-5-carbonsäurederivat der Formel I, gemäss Anspruch 1 enthält.

11. Mittel gemäss Anspruch 10, dadurch gekennzeichnet, dass es zwischen 0,1 % und 95 % Wirkstoff der Formel I gemäss Anspruch 1 enthält.

12. Verfahren zur Hemmung des Pflanzenwachstums, dadurch gekennzeichnet, dass man einen Wirkstoff der Formel I, gemäss Anspruch 1 oder ein diesen Wirkstoff enthaltendes Mittel in einer wirksamen Menge auf die Pflanzen oder deren Lebensraum appliziert.

13. Verfahren gemäss Anspruch 12, dadurch gekennzeichnet, dass man eine Wirkstoffmenge zwischen 0,01 und 10 kg pro Hektar appliziert.